# EUROPEAN PATENT SPECIFICATION

(11) **EP 3 148 630 B1**
(45) Date of publication and mention of the grant of the patent: **07.12.2022**
(21) Application number: 14893532.3
(22) Date of filing: 28.05.2014
(51) Int. Cl.: A61M 37/00, A61K 9/00

(54) **A FLEXIBLE DISSOLVABLE PATCH AND ITS METHOD OF FABRICATING**
FLEXIBLES LÖSLICHES PFLASTER UND DESSEN HERSTELLUNGSVERFAHREN
TIMBRE SOLUBLE SOUPLE ET SON PROCÉDÉ DE FABRICATION

(43) Date of publication of application: 05.04.2017
(73) Proprietor: MDAPP S.R.L., 25124 Brescia (BS) (IT)
(72) Inventor: LIM, Chee Yen, Singapore 681683 (SG); CHIA, Yong Wei, Singapore 542323 (SG); LEE, Wei Siong, Singapore 730755 (SG)
(74) Representative: Biggi, Cristina
(86) International application number: PCT/SG2014/000231
(87) International publication number: WO 2015/183179

(56) References cited:
- EP-A1- 2 213 284
- WO-A1-2009/142741
- WO-A1-2014/041531
- CN-A- 102 499 667
- JP-A- 2006 341 089
- US-A1- 2008 245 764
- US-A1- 2010 228 203
- US-A1- 2011 237 925
- US-A1- 2014 005 606

## Description

### BACKGROUND

### Field of the Invention

The present invention relates to a flexible dissolvable patch comprising dissolvable microneedles for delivering targeted substances to the skin painlessly. Particularly, the present invention relates to a wearable patch that conforms to body contours, rapidly delivers drugs or dissolving substances to the skin, and seals the drug in the skin to prevent it from coming off the skin, from back diffusion into the substrate or contamination.

### Background of the Invention

Painless intradermal injection can be achieved by new technologies such as microneedle technology. It has been reported that microneedles were used to deliver vaccines, hormones and drugs to the animal skin with excellent pharmacodynamics results. Recently, there have been microneedle products made for cosmetic use. These microneedle products are used for creating micro-wounds so that allergens can be absorbed by the skin through healing, or delivering cosmetic ingredients such as chitosan, hydrogels and hyaluronic acids to the superficial layer of the skin for rejuvenating the skin.

A critical issue in dissolvable microneedles is the dissolving time. In Cosmed's patent 8,167,852B2, the dissolvable material used is hyaluronic acid (HA), which has molecular weight of 5,000 - 20,000,000 Da. Long duration is required for the HA to dissolve in the skin after penetration. The typical dissolving duration required for HA is half an hour to an hour, which may be too long for most cosmetic and medical applications. Another long-duration example is provided by US patent 8,708,966B2, awarded to Georgia Tech Research Corporation, which uses biodegradable polymers such as polylactide and polyglicolide, which may degrade over a few weeks or months. The cosmetic applications include facial mask and facial patches, and the medical applications include hypersensitivity diagnosis and treatment, vaccination, and allergic skin testing. Hence, rapid dissolution of the microneedles is highly desirable.

Another critical issue for such microneedle patch applications is that wearing such patches requires the patches to be flexible to conform to body contours and curvatures, particularly on the face, neck, upper arms and wrists. Currently, all dissolvable microneedles are made of hard or in-flexible materials, namely chitosans and hyaluronic acids, which cannot conform to body contours. Such materials are used so that the microneedles are strong enough to penetrate the skin effectively. In conclusion, a wearable microneedle patch that is flexible for conforming to the body contour is desired.

US patents 8,491,534B2 and 8,506,980B2 awarded to Bioserentach provides a carrier sheet for holding the microneedles on its surface using adhesive substance on coated on the sheet.

This idea is not feasible because adhesive is not permanent and the tiny microneedles may dislodge from the sheet prior or during the use of the patch.

The last and the most critical issue of current dissolvable microneedle patch applications is that after the drug loaded on the microneedles is delivered by inserting the dissolvable microneedles into the skin, there is no sealing of the skin to allow the drug to be slowly absorbed or metabolized by the skin. The absorption or the metabolism of the drug and the dissolvable microneedles by the skin requires a length of finite time and during this time the drug may either come off from the skin or may diffuse into the base layer (or hereinafter the 'substrate' or the 'flexible substrate'). Currently, all the dissolvable microneedle patches have a substrate that is made of the same material as the dissolvable microneedles and the drug is risked to diffuse into the base layer during the application time (or wear time) or it is risked to come off of the skin after the removal of the patch. It is therefore highly preferable that the substrate of the dissolvable microneedles is not only flexible, but also non-dissolving and adheres to the skin to provide a sealing to allow the drug to be absorbed or metabolized completely by the skin.

WO 2014/041531 discloses an applicator comprising a substrate, an array of microneedles and a skin augmentation composition useful for augmenting skin, filling undesired lines, wrinkles, depressed scars and folds in a subject. The microneedles comprise: - a skeleton made of a rigid material (e.g. metal, plastic, ceramic, silicone) comprising a sharp tip to penetrate a skin of a subject, a base and a middle section connecting between the sharp tip and the base, optionally the middle section can be inserted with an extension into the substrate; - a skin augmentation composition which at least partially surrounds the middle section of the microneedle skeleton, such that a diameter of the sharp tip section is larger than a diameter of the augmentation composition surrounding the skeleton of the microneedle. Said skin augmentation composition comprises at least one biocompatible ceramic material (e.g. calcium phosphate ceramic material, hydroxyapatite etc.) that is injected into the dermis layer of a subject's skins and remains there for a prolonged time-period. The skin augmentation composition can also comprise a biodegradable carrier, at least one type of skin augmentation material (e.g. hyaluronic acid) and a biological active agent.

There is a need for rapid dissolving, self-sealing and body conforming microneedle patches wherein the microneedles are permanently fastened onto the patch for cosmetic and medical applications. The present invention seeks to provide a solution to this need.

### SUMMARY OF THE INVENTION

The present invention aims to provide a solution to the current problematic microneedle devices which are deficient in various aspects as discussed in the previous section. In particular, the present invention seek to provide solutions in three aspects: (1) to provide a rapid dissolving microneedle patches so that the wear time can be minimized; (2) to provide a flexible substrate so that the dissolvable microneedles can conform to body contours and curvatures and be worn on body parts such as face, arms, and wrists; lastly (3) to provide a self-sealing non-dissolving flexible substrate which prevents the delivered drug to come off the skin or to diffuse into the substrate.

According to the invention there is provided a flexible dissolvable patch, comprising: a. A plurality of dissolvable microneedles made from a dissolving and hard material chosen among chitosans, hyaluronic acids and polysaccharides; and b. A flexible substrate made from a non-dissolving and soft material, wherein the plurality of dissolvable microneedles further comprising at least an undercut feature which is disposed at the base portions of each individual microneedles, such that the dissolvable microneedles are fastened onto the flexible substrate via the undercut features.

Further, according to the invention there is provided a method of fabricating said flexible dissolvable patch, the method comprising: a. Providing a microneedle template comprising a plurality of microneedle cavities and a substrate cavity; b. Providing a flexible substrate comprising a plurality of undercut features; c. disposing the soft flexible substrate in the substrate cavity on the microneedle template such that the undercut features are aligned to the microneedle cavities, thereby forming an integrated template; d. forming a plurality of dissolvable microneedles having undercut features on their base portions by casting, then solidifying a dissolving and hard material in the plurality of microneedle cavities on the integrated template; and e. de-moulding the flexible dissolvable patch from the microneedle template.

The advantages of the present invention is clear to for complementing the current long-felt needs of microneedle devices, which include (1) the conformance ability to body contours while penetrating the skin and delivering a substance effectively and rapidly, (2) the undercut features of the dissolvable microneedles providing a fastening means for holding the microneedles firmly on the flexible substrate, (3) a natural tacky substrate to provide firm but temporary adhesion for self-sealing so that the drugs or the dissolving substances do not come off from the skin after the patch removal, and (4) a flexible and non-dissolving substrate which prohibits back diffusion of the drugs or dissolving substances from the dissolvable microneedles into the substrate. Lastly, yet another advantage of the present invention is the cost effective method in fabricating the flexible-based dissolvable microneedle patches.

### BRIEF DESCRIPTIONS OF THE DRAWINGS

Figure 1 discloses prior art. Figures 2 to 4 and 9 disclose comparative not claimed examples.
FIG. 1 shows a general overview of a flexible dissolvable patch.
FIG. 2 shows cross-sectional views of a flexible dissolvable patch.
FIG. 3 shows a schematic diagram of the actuation of a flexible dissolvable patch on a target skin site.
FIG. 4 shows a cross-sectional view of a flexible dissolvable patch, wherein a thin layer of carrier sheet is encapsulated in the flexible substrate of the device.
FIG. 5 shows a cross-sectional view of a flexible dissolvable microneedle with undercut features.
FIG. 6 shows different types of undercut features in a flexible dissolvable patch.
FIG. 7 shows a cross-sectional view of the surfaces of a flexible dissolvable patch that are in contact with an applicator or the fingers of a subject.
FIG. 8 shows a schematic diagram illustrating the compression and reaction forces acting on a flexible dissolvable patch with undercut features when it is actuated onto a target skin site.
FIG. 9 shows a schematic diagram illustrating the fabrication process of a flexible dissolvable patch.
FIG. 10 shows a schematic diagram illustrating the fabrication process of a flexible dissolvable patch with undercut features.

### DETAILED DESCRIPTION OF THE INVENTION

### Prior Art

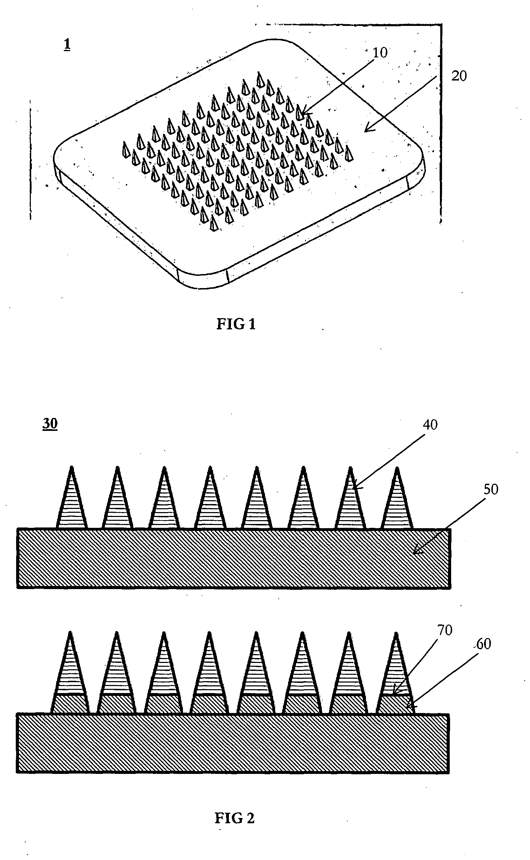

FIG. 1 shows a dissolvable microneedle device 1, comprising a plurality of dissolvable microneedles 10 and a flexible substrate 20. Typically, a flexible dissolvable patch 1, which includes the plurality of dissolvable microneedles 10 and the substrate 20, both are made of same polymeric substances, such as saccharides, proteins, hydrogels and synthetic polymers, which are non-toxic and biodegradable in nature. The dissolvable microneedles 10 may be used to encapsulate and deliver cosmetic or active pharmaceutical ingredients (API) for transdermal drug delivery when they penetrate and are implanted into the skin of a subject. The encapsulated drugs may exist only in a section in the dissolvable microneedle body or it may exist in the entire body of a dissolvable microneedle **10.** Such a device is very common in the prior art. Since the materials used has to provide sufficient mechanical strength such as hardness and toughness for effective skin penetration by the dissolvable microneedles **10,** the substrate **20** may be too hard and too rigid to conform to body contours and curvatures. Moreover, the dissolvable microneedle patches do not provide a sealing means to insulate the drugs or the dissolving substances that are delivered in the skin. As a consequence, the drugs or the dissolving substances that are delivered into the skin may come off from the skin. There is also a hazard of contamination from the environment if the applied skin site is not sealed. Another very serious problem is that for such configuration, it is possible for the API to diffuse from the dissolvable microneedles to the substrate thereby varying the final dose of the API. This defect is also wastage where precious API remains un-delivered to the skin. The present invention aims to to solve these several or all problems in several embodiments provided herein.

### Flexible dissolvable patch (figures 2 to 4 examples not claimed)

**FIG. 2** shows an example, wherein the flexible dissolvable patch **30** comprises a plurality of dissolvable microneedles **40** that is disposed onto a top surface (not shown) of the flexible substrate **50.** The materials for making dissolvable microneedles **40** include but not limited to various dissolving materials such as chitosans, hyaluronic acids, hydrogels, polysaccharides, biodegradable materials, and so on. It is discovered by the inventors that using these materials with small molecular weight will meaningfully increase the dissolving rate. The small molecular weight may be ranging from 1 to 20,000 Da, or more particularly, from 3,000 Da to 10,000 Da.

The flexible substrate **50** in **FIG. 2** is made of non-dissolving and soft materials, such as but not limited to silicone-based polymers and thin layered plastics. Examples of silicone-based polymers include poly-dimethyl-siloxane (PDMS) and room temperature vulcanizing silicone (RTV), whereas thin layered plastics can include polycarbonate and polyurethane. Particularly, a tacky silicone gel is an ideal material for making the flexible substrate **50** because it naturally leaves a tacky (adhesive) surface when cured and this natural tacky surface provides a strong but temporary adhesion to the skin. The materials for making the flexible substrate **50** has to provide three functions: (1) to provide flexibility for conformance to body contours and curvatures; (2) to provide self-sealing so that the drug or the dissolving substance in the dissolvable microneedles are isolated from the environment to prevent possible contamination or the drug coming off from the skin; and (3) to prevent back diffusion of the drugs or dissolving substance from the dissolvable microneedles into the flexible substrate.

The flexible substrate **50** acts as a platform to hold and fixate the dissolvable microneedles **40** on the substrate, so that a force can be applied on the flexible substrate to insert the dissolvable microneedles **40** into the skin for delivering drug transdermally or intradermally. With this flexible substrate **50,** the flexible dissolvable patch **30** will be able to conform to different body contours and curvatures, particularly the face, neck, upper arms and wrists. The plurality of dissolvable microneedles **40** may extend from a top surface of the flexible substrate **50,** or they may extend from a plurality of base interfaces **70** on a plurality of microneedle bases **60** which extend from the flexible substrate **50.** The dissolvable microneedles **40** can be fastened to the flexible substrate **50** via the base interfaces **70** using passive adhesion or UV covalent bonding or any other suitable fastening means.

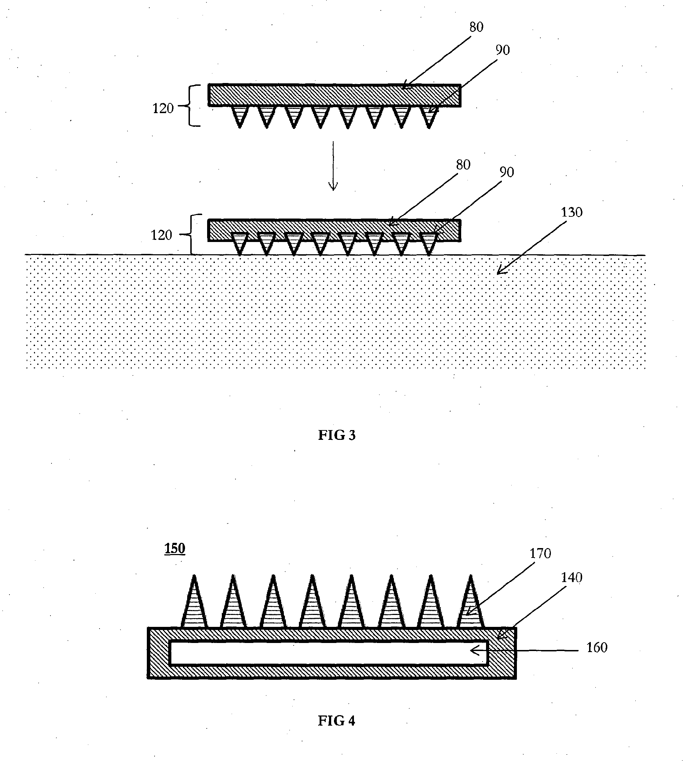

**FIG. 3** shows how a flexible dissolvable patch **120** is applied to a skin site **130.** One skilled in the art will recognize that the material hardness of the flexible substrate **80** of a flexible dissolvable patch plays a crucial role in the ability of the dissolvable microneedles **90** to penetrate the skin of a subject during transdermal drug delivery. When the hardness of the flexible substrate **80** is less than Shore A 20, the plurality of dissolvable microneedles **90** will not be able to penetrate consistently into the skin because the dissolvable microneedles will sink and collapse into the flexible substrate. This is further explained where the flexible substrate **80** is unable to provide sufficient resistance force to keep the dissolvable microneedles **90** fixated in place, leading to incomplete drug delivery. Therefore, it is preferable for the material hardness of the flexible substrate **80** to be more than hardness Shore A 20. The materials for making dissolvable microneedles **90** include but not limited to various dissolving materials such as chitosans, hyaluronic acids, hydrogels, polysaccharides, biodegradable materials, and so on. It is discovered by the inventors that using these materials with small molecular weight will meaningfully increase the dissolving rate. The small molecular weight may be ranging from 1 to 20,000 Da, or more particularly, from 3,000 Da to 10,000 Da.

The flexible substrate **80** in **FIG. 3** is made of non-dissolving and soft materials, such as but not limited to silicone-based polymers and thin layered plastics. Examples of silicone-based polymers include poly-dimethyl-siloxane (PDMS) and room temperature vulcanizing silicone (RTV), whereas thin layered plastics can include polycarbonate and polyurethane. Particularly, a tacky silicone gel is an ideal material for making the flexible substrate **80** because it naturally leaves a tacky (adhesive) surface when cured and this natural tacky surface provides a strong but temporary adhesion to the skin.

**FIG. 4** shows another example. In this example, a thin layer of carrier sheet **160,** such as metals or plastics, is embedded within the flexible substrate **140** to enhance the rigidity of the flexible substrate **140.** The thickness of the carrier sheet **160** may range from 0.05mm to 2mm, where applicable. The carrier sheet **160** acts as a resistance platform that prevents the dissolvable microneedles from retracting into the flexible substrate **140.** This is very useful for a flexible substrate that requires a material which is softer than hardness Shore A 20 due to skin compatibility, comfort or other cosmetic reasons.

### Flexible dissolvable patch with undercut features

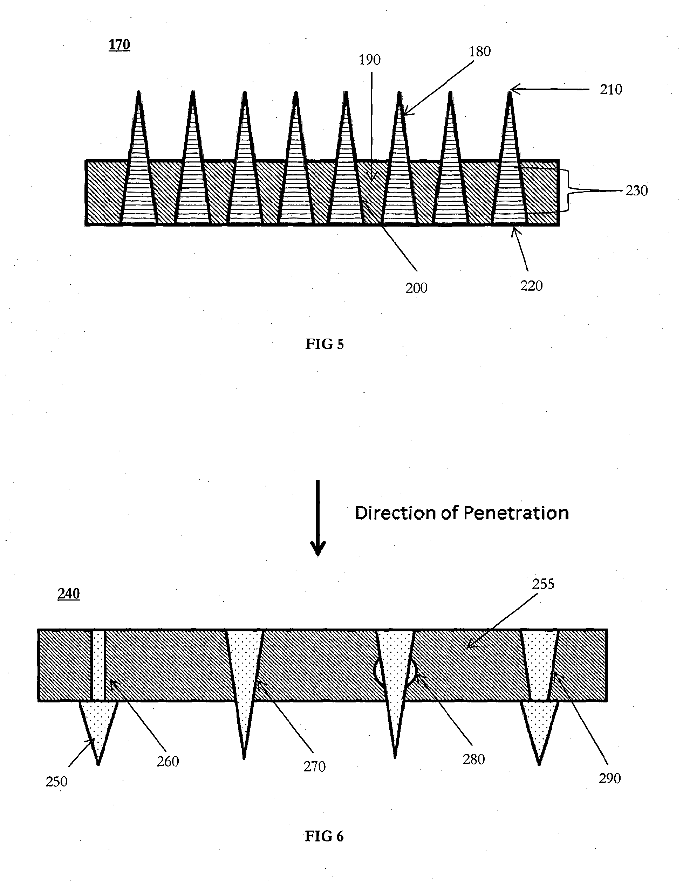

**FIG. 5** shows an embodiment of the present invention. In this embodiment, the flexible dissolvable patch **170** comprises a plurality of dissolvable microneedles **180,** each dissolvable microneedle having an undercut feature **200,** a flexible substrate **190,** wherein the plurality of dissolvable microneedles are fastened onto the flexible substrate **190** by means of undercut features **200.** The dissolvable microneedles **180** extend from the microneedle apex **210** to the microneedle base **220,** and the undercut features **200** is disposed at the base portion **230** of the dissolvable microneedle, which is within the thickness of the flexible substrate **190.** In this example, the undercut feature is a tapering extension of the dissolvable microneedles. However, this tapering geometry is only one example of the undercut feature that can be used in our embodiment; any other undercut features that are able to provide a fastening means are included in our embodiment. The materials for making dissolvable microneedles **180** include but not limited to various dissolving materials such as chitosans, hyaluronic acids, hydrogels, polysaccharides, biodegradable materials, and so on. It is discovered by the inventors that when these materials are composed with small molecules, the dissolving rate is meaningfully increase. The range of molecular weight that is suitable for our applications is less than 20,000 Da, or more particularly, between 3,000 Da to 10,000 Da.

The flexible substrate **190** in **FIG. 3** is made of non-dissolving and soft materials, such as but not limited to silicone-based polymers and thin layered plastics. Examples of silicone-based polymers include poly-dimethyl-siloxane (PDMS) and room temperature vulcanizing silicone (RTV), whereas thin layered plastics can include polycarbonate and polyurethane. Particularly, a tacky silicone gel is an ideal material for making the flexible substrate **190** because it naturally leaves a tacky (adhesive) surface when cured and this natural tacky surface provides a strong but temporary adhesion to the skin.

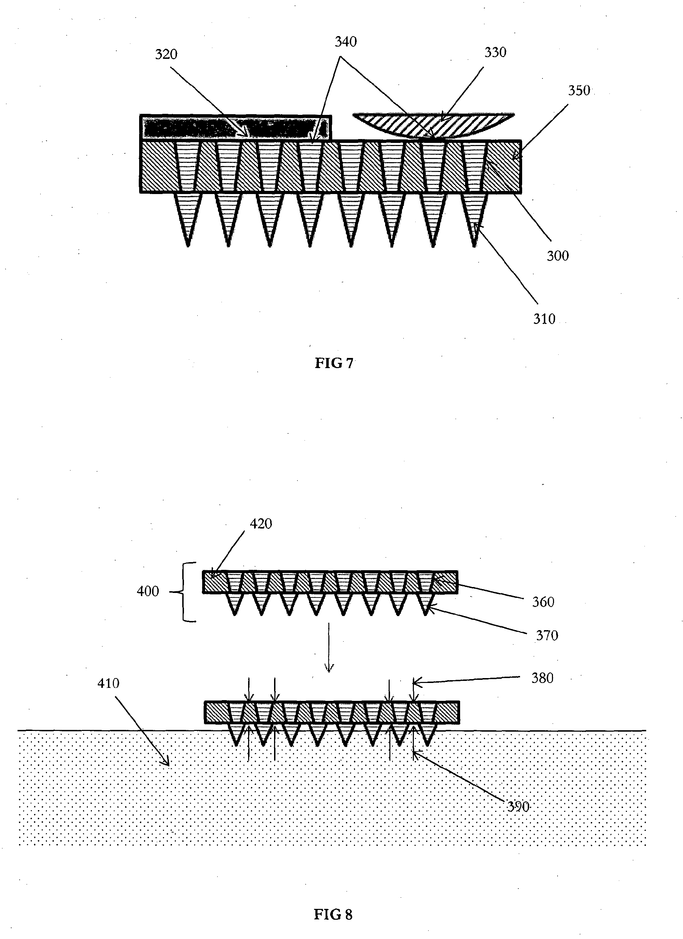

**FIGS. 6 - 8** show a limited number of possible undercut features that are effective as a fastening means for affixing the dissolvable microneedles to the flexible substrate. **FIG. 6** shows a flexible dissolvable patch **240** comprising a number of undercut features **260, 270, 280 and 290** that are effective in fastening the dissolvable microneedles **250** to the flexible substrate **255.** The major consideration in the fastening of the undercut features **260** - **290** is that the dissolvable microneedles **250** may come off or dislodge from the flexible substrate **255** before they are applied to the skin, for instance during a manufacturing process, transportation or storage handling. It is vital that an undercut feature is able to provide sufficient fastening strength so that the dissolvable microneedles **250** are firmly affixed to the flexible substrate **255** prior to use. In addition, the undercut features are supposed to be strong enough to sustain compression and reaction pressures on and from the skin, as illustrated in **FIGS. 7 - 8****.**

**FIG. 7** shows a schematic diagram of a flexible dissolvable patch with undercut features being applied to the skin. The presence of the undercut features **300** resists the compression force coming from the back of the dissolvable microneedles **310,** which may be exerted by an actuator **320** or by a finger **330,** so that they will not be pushed out from the flexible substrate **350.** The undercut features **300** which extend from the bottom surface **340** to the apex of the dissolvable microneedles (not shown) transmit the compression force by the actuator **320** or by the finger **330** from the back surface **340** to the apex of the dissolvable microneedles so that skin penetration can be achieved effectively.

**FIG. 8** shows a schematic diagram with the compression force **380** and reaction force **390** exerted on the flexible dissolvable patch **400** during a skin penetration procedure. It can be seen from the diagram clearly that the undercut features **360** form an interlocking means between the dissolvable microneedles **370** and the flexible substrate **420,** thereby preventing the dissolvable microneedles **370** from dislodging from their positions by either the compression force **380** or the reaction forces **390** when the flexible dissolvable patch **400** is pressed against a target skin site **410.**

### Fabrication method of flexible dissolvable patch

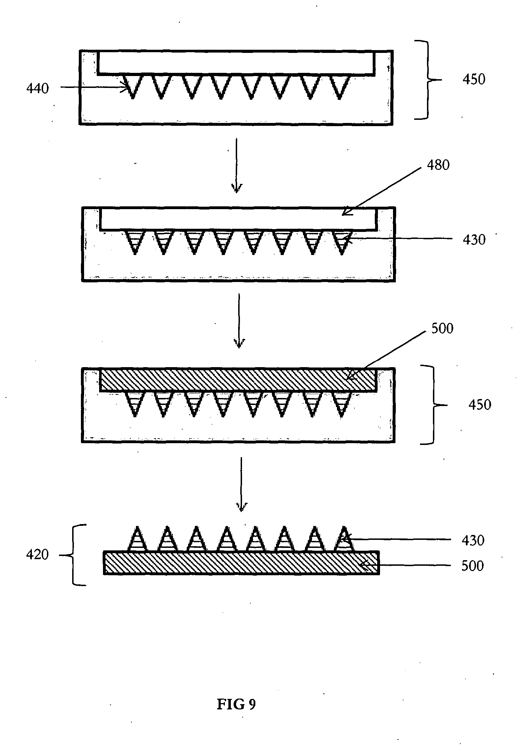

**FIG. 9** shows a flow chart of a manufacturing process. First, a microneedle template **450** comprising a plurality of microneedle cavities **440** and a substrate cavity **480** is provided. Subsequently, a first polymeric solution is deposited on the plurality of microneedle cavities **440.** A centrifugation or vacuuming process is used for ensuring that the polymeric solution completely fills-up the microneedle cavities **440.** Then, the polymeric solution is left to solidify in the microneedle cavities 440 via dehydration, vacuuming, centrifugation or other methods known by a person skilled in the art in a humidity-controlled environment. After the polymeric solution has completely solidified, a plurality of dissolvable microneedles **430** is formed in the microneedle cavities **440.** The materials for the first polymeric solution include chitosans, hyaluronic acids, hydrogels, polysaccharides, biodegradable materials and so on and so forth. It is discovered by the inventors that when these materials are composed with small molecules, the dissolving rate is meaningfully increase. The range of molecular weight that is suitable for our applications is less than 20,000 Da, or more particularly, between 3,000 Da to 10,000 Da. Subsequently, a second polymeric solution is deposited on the microneedle template **450** until the substrate cavity **480** is partially or completely filled-up with the second polymeric solution. A de-gassing step via centrifugation, sonication or vacuuming may be required to remove any air bubbles. After the second polymeric solution is cured or solidified, a flexible substrate **500** is finally formed. Lastly, de-moulding of the solidified product is performed and a flexible dissolvable patch **420** is realized. The materials for the second polymeric solution include self-curing polymers such as silicone-based organic polymers with low viscosity and high rheological properties. Particularly, a tacky silicone gel is very much suited for making the flexible substrate **500.** Such tacky silicone gel when cured provides a good adhesive surface for properly sealing the drugs or dissolving substance in the skin and preventing them from accidental coming off from the skin or contamination. More importantly, the tacky silicone gel is a non-dissolving material which prohibits the back diffusion of the drugs or dissolving substance from the dissolvable microneedles.

It is straightforward for a person skilled in the art to make the flexible substrate **500** further embed a carrier sheet for providing rigidity to the patch **420.**

### Fabrication of flexible dissolvable patch with undercut features

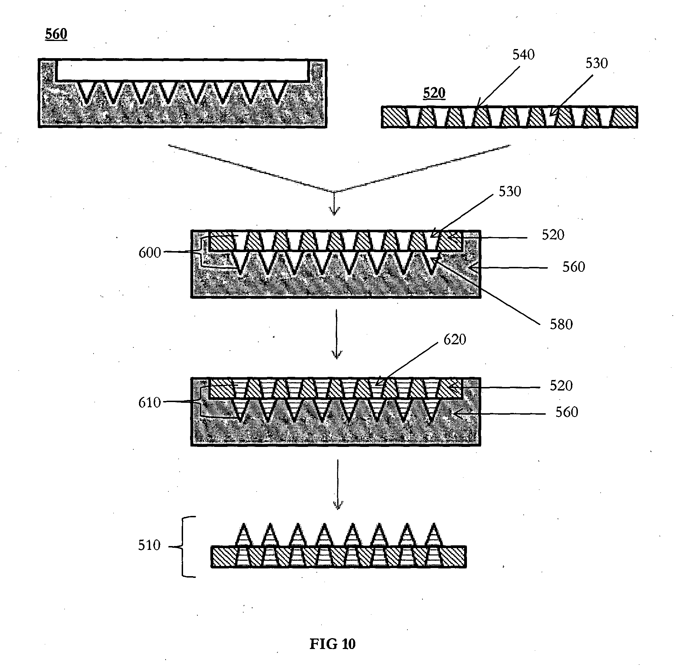

The present invention also provides for a method to make the flexible dissolvable patch. **FIG. 10** shows a flow chart of an embodiment of making a flexible dissolvable patch. A flexible dissolvable patch with undercut features **540** is fabricated by first providing a flexible substrate **520,** comprising a plurality of through holes **530** with undercut features **540,** as shown in **FIG. 10****.** The flexible substrate **520** is disposed into a microneedle template **560** such that each through-hole **530** of the flexible substrate **520** is aligned to each microneedle cavity **580** within the microneedle template **560.** This leads to an integrated template, where a plurality of integrated cavities **600,** which are made up of a plurality of through holes **530** on the flexible substrate **520** and a plurality of microneedle cavities **580** on the microneedle template **560,** are formed. A polymeric solution is deposited onto the flexible substrate **520** and is made to fill-up the plurality of integrated cavities **600** by vacuuming, centrifugation and other methods known by a person skilled in the art. Once the polymeric solution completely fills-up the integrated cavities **600,** the polymeric solution is left to solidify in the integrated cavities **600** via vacuuming, centrifugation, or air-drying in a humidity-controlled environment until it is completely cured or solidified. A plurality of dissolvable microneedles **610** with undercut features **620** is formed. Lastly, a de-moulding process will remove the product from the microneedle template **560** and a new flexible dissolvable patch **510** is realized.

## Claims

1. A flexible dissolvable patch, comprising:
a. A plurality of dissolvable microneedles (180) made from a dissolving and hard material chosen among chitosans, hyaluronic acids and polysaccharides; and
b. A flexible substrate (190) made from a non-dissolving and soft material, wherein the plurality of dissolvable microneedles (180) further comprising at least an undercut feature (200) which is disposed at the base portions of each individual microneedles (180), such that the dissolvable microneedles (180) are fastened onto the flexible substrate (190) via the undercut features (200).

2. A flexible dissolvable patch according to claim 1, wherein the flexible substrate (190) is made from a tacky silicone gel so that the flexible substrate (190) has an adhesive surface for adhering to the skin firmly and temporarily.

3. A flexible dissolvable patch according to claim 1, wherein the plurality of dissolvable microneedles (180) encapsulate cosmetic or active pharmaceutical ingredients in at least one region or all parts of each dissolvable microneedle.

4. A flexible dissolvable patch according to claim 1, wherein the dissolving and hard substances include chitosans, hyaluronic acids, saccharides, proteins, hydrogels and synthetic polymers have molecular weight less than 10,000 Da.

5. A method of fabricating a flexible dissolvable patch according to claim 1, comprising:
a. Providing a microneedle template (560) comprising a plurality of microneedle cavities (580) and a substrate cavity (480);
b. Providing a flexible substrate comprising a plurality of undercut features;
c. disposing the soft flexible substrate in the substrate cavity (480) on the microneedle template (560) such that the undercut features are aligned to the microneedle cavities (580), thereby forming an integrated template;
d. forming a plurality of dissolvable microneedles having undercut features on their base portions by casting, then solidifying a dissolving and hard material in the plurality of microneedle cavities on the integrated template; and
e. de-moulding the flexible dissolvable patch from the microneedle template.

6. A method of fabricating a flexible dissolvable patch according to claim 5, wherein the dissolving and hard substances include chitosans, hyaluronic acids, saccharides, proteins, hydrogels and synthetic polymers.

7. A method of a fabricating flexible dissolvable patch according to claim 5, wherein the dissolvable microneedles are solidified by vacuuming, centrifugation or dehydration processes in a humidity-controlled environment.

8. A method of fabricating a flexible dissolvable patch according to claim 5, wherein the undercut features of the patch are located within the flexible substrate.

9. A method of fabricating flexible dissolvable patches in claim 5, wherein the flexible substrate is fabricated using non-dissolving and soft materials, preferably including self-curing silicone-based polymers or plastics.

## Patentansprüche

1. Flexibles, auflösbares Pflaster, umfassend:
a. eine Vielzahl von auflösbaren Mikronadeln (180) aus einem auflösbaren und harten Material, das aus Chitosanen, Hyaluronsäuren und Polysacchariden ausgewählt ist; und
b. Ein flexibles Substrat (190), das aus einem nicht-auflösenden und weichen Material hergestellt ist, wobei die Vielzahl von auflösbaren Mikronadeln (180) ferner mindestens ein Hinterschneidungsmerkmal (200) umfasst, das an den Basisabschnitten jeder einzelnen Mikronadel (180) angeordnet ist, so dass die auflösbaren Mikronadeln (180) über die Hinterschneidungsmerkmale (200) an dem flexiblen Substrat (190) befestigt sind.

2. Flexibles auflösbares Pflaster nach Anspruch 1, wobei das flexible Substrat (190) aus einem klebrigen Silikongel hergestellt ist, so dass das flexible Substrat (190) eine Klebefläche aufweist, um fest und vorübergehend an der Haut zu haften.

3. Flexibles auflösbares Pflaster nach Anspruch 1, wobei die Vielzahl von auflösbaren Mikronadeln (180) kosmetische oder pharmazeutische Wirkstoffe in mindestens einem Bereich oder allen Teilen jeder auflösbaren Mikronadel einkapseln.

4. Flexibles auflösbares Pflaster nach Anspruch 1, wobei die auflösbaren und harten Substanzen Chitosane, Hyaluronsäuren, Saccharide, Proteine, Hydrogele und synthetische Polymere mit einem Molekulargewicht von weniger als 10.000 Da umfassen.

5. Verfahren zur Herstellung eines flexiblen, auflösbaren Pflasters nach Anspruch 1, umfassend:
a. Bereitstellen einer Mikronadelschablone (560), die eine Vielzahl von Mikronadelhohlräumen (580) und einen Substrathohlraum (480) umfasst;
b. Bereitstellen eines flexiblen Substrats mit einer Vielzahl von Hinterschneidungsmerkmalen;
c. Anordnen des weichen, flexiblen Substrats in dem Substrathohlraum (480) auf der Mikronadelschablone (560), so dass die hinterschnittenen Merkmale auf die Mikronadelhohlräume (580) ausgerichtet sind, wodurch eine integrierte Schablone gebildet wird;
d. Ausbilden einer Vielzahl von auflösbaren Mikronadeln mit hinterschnittenen Merkmalen an ihren Basisabschnitten durch Gießen und anschließendes Verfestigen eines auflösbaren und harten Materials in der Vielzahl von Mikronadelhohlräumen auf der integrierten Schablone; und
e. Entformen des flexiblen, auflösbaren Pflasters von der Mikronadelschablone.

6. Verfahren zur Herstellung eines flexiblen, auflösbaren Pflasters nach Anspruch 5, wobei die auflösenden und harten Substanzen Chitosane, Hyaluronsäuren, Saccharide, Proteine, Hydrogele und synthetische Polymere umfassen.

7. Verfahren zur Herstellung eines flexiblen auflösbaren Pflasters nach Anspruch 5, wobei die auflösbaren Mikronadeln durch Vakuum-, Zentrifugations- oder Dehydrationsverfahren in einer feuchtigkeitskontrollierten Umgebung verfestigt werden.

8. Verfahren zur Herstellung eines flexiblen, auflösbaren Pflasters nach Anspruch 5, wobei die hinterschnittenen Merkmale des Pflasters innerhalb des flexiblen Substrats angeordnet sind.

9. Verfahren zur Herstellung von flexiblen auflösbaren Pflastern nach Anspruch 5, wobei das flexible Substrat unter Verwendung von nicht auflösenden und weichen Materialien, vorzugsweise einschließlich selbsthärtender Polymere oder Kunststoffe auf Silikonbasis, hergestellt wird.

## Revendications

1. Un patch flexible dissoluble, comprenant :
a. Une pluralité de micro-aiguilles dissolvables (180) réalisées à partir d'un matériau dissolvant et dur choisi parmi les chitosanes, les acides hyaluroniques et les polysaccharides ; et
b. Un substrat flexible (190) fait d'un matériau non dissolvant et mou, dans lequel la pluralité de micro-aiguilles dissolvables (180) comprend en outre au moins une caractéristique de contre-dépouille (200) qui est disposée au niveau des parties de base de chaque micro-aiguille individuelle (180), de sorte que les micro-aiguilles dissolvables (180) sont fixées sur le substrat flexible (190) via les caractéristiques de contre-dépouille (200).

2. Patch flexible dissoluble selon la revendication 1, dans lequel le substrat flexible (190) est fait d'un gel de silicone collant de sorte que le substrat flexible (190) a une surface adhésive pour adhérer à la peau fermement et temporairement.

3. Patch flexible dissoluble selon la revendication 1, dans lequel la pluralité de micro-aiguilles dissolubles (180) encapsule des ingrédients cosmétiques ou pharmaceutiques actifs dans au moins une région ou toutes les parties de chaque micro-aiguille dissoluble.

4. Patch flexible dissoluble selon la revendication 1, dans lequel les substances dissolvantes et dures comprennent des chitosanes, des acides hyaluroniques, des saccharides, des protéines, des hydrogels et des polymères synthétiques ont un poids moléculaire inférieur à 10 000 Da.

5. Procédé de fabrication d'un patch dissoluble flexible selon la revendication 1, comprenant :
a. Fournir un modèle de micro-aiguille (560) comprenant une pluralité de cavités de micro-aiguille (580) et une cavité de substrat (480) ;
b. Fournir un substrat souple comprenant une pluralité de caractéristiques de contre-dépouille;
c. disposer le substrat souple dans la cavité de substrat (480) sur le gabarit de micro-aiguille (560) de sorte que les caractéristiques de contre-dépouille soient alignées sur les cavités de micro-aiguille (580), formant ainsi un gabarit intégré ;
d. former une pluralité de micro-aiguilles dissolvables ayant des caractéristiques de contre-dépouille sur leurs parties de base par moulage, puis solidifier un matériau dissolvant et dur dans la pluralité de cavités de micro-aiguilles sur le modèle intégré ; et
e. démouler le timbre souple dissoluble du modèle de micro-aiguilles.

6. Procédé de fabrication d'un timbre souple dissoluble selon la revendication 5, dans lequel les substances dissolvantes et dures comprennent des chitosanes, des acides hyaluroniques, des saccharides, des protéines, des hydrogels et des polymères synthétiques.

7. Procédé de fabrication d'un timbre souple dissolvable selon la revendication 5, dans lequel les micro-aiguilles dissolvables sont solidifiées par des procédés de mise sous vide, de centrifugation ou de déshydratation dans un environnement à humidité contrôlée.

8. Procédé de fabrication d'un patch flexible dissolvable selon la revendication 5, dans lequel les caractéristiques de contre-dépouille du patch sont situées à l'intérieur du substrat flexible.

9. Procédé de fabrication de patchs dissolvables flexibles selon la revendication 5, dans lequel le substrat flexible est fabriqué en utilisant des matériaux non dissolvants et mous, comprenant de préférence des polymères ou des plastiques à base de silicone autodurcissables.
